# EUROPÄISCHE PATENTSCHRIFT

(11) **EP 2 482 765 B1**
(45) Veröffentlichungstag und Bekanntmachung des Hinweises auf die Patenterteilung: **15.03.2017**
(21) Anmeldenummer: 10774127.4
(22) Anmeldetag: 01.10.2010
(51) Int. Cl.: A61F 2/954, A61F 2/958, A61F 2/07, A61M 25/10

(54) **SYSTEM BESTEHEND AUS BALLONKATHETER UND STENTGRAFT ZUR PLATZIERUNG IN EINER GEFÄSSBIFURKATION**
SYSTÈME COMPRENANT UN CATHETER À BALLONNET ET GREFFE D'ENDOPROTHÈSE& xA;POUR LE PLACEMENT DANS UN VAISSEAU BIFURQUÉ
SYSTEM COMPRISING A BALLOON CATHETER AND STENT-GRAFT FOR PLACEMENT IN A BIFURCATION

(30) Priorität: 01.10.2009 DE 102009047925
(43) Veröffentlichungstag der Anmeldung: 08.08.2012
(73) Patentinhaber: Qualimed Innovative Medizinprodukte GmbH, 21423 Winsen (DE)
(72) Erfinder: GÜLCHER, Manfred, 46348 Raesfeld-Erle (DE)
(74) Vertreter: Isfort, Olaf
(86) Internationale Anmeldenummer: PCT/EP2010/005998
(87) Internationale Veröffentlichungsnummer: WO 2011/038928

(56) Entgegenhaltungen:
- EP-A1- 0 747 088
- WO-A1-90/15582
- US-A1- 2007 142 819
- US-A1- 2007 173 920

## Beschreibung

Die Erfindung betrifft ein System zur Platzierung von Stentgrafts mit einem proximalen und zwei distalen Ballonen, einem Multilumenschaft mit einem Lumen für die Aufnahme eines Führungsdrahts und wenigstens zwei Lumina zur individuellen Beaufschlagung der Ballone mit hydraulischem Druck.

Aneurysmen sind Ausweitungen der Blutgefäße, die mit einer Ausdünnung der Gefäßwand einhergehen. Es kann sich dabei um angeborene oder erworbene Gefäßwandveränderungen handeln. Bei stark ausgedünnter Gefäßwandung droht unter dem Druck des anfließenden Blutes eine Ruptur, die schwere gesundheitliche Nachteile oder den Tod nach sich ziehen kann. Dies gilt insbesondere für Aneurysmen im zerebralen Bereich sowie Aneurysmen der Schlagadern, insbesondere der Aorta im Brust- und Bauchraum.

Die Behandlung eines bestehenden Aneurysmas im Brust- oder Bauchraum erfolgt zumeist auf operativem Weg durch Einnähen einer Gefäßprothese aus Dacron oder PTFE (Polytetrafluorethylen), wobei auf abgehende Gefäße Rücksicht genommen werden muss. Die operative Behandlung ist mit einem nicht unerheblichen Letalitätsrisikö behaftet.

Insbesondere im zerebralen Bereich werden Aneurysmen endovaskulär behandelt, wobei mittels eines Katheters Wendeln aus einem Edelmetalldraht im Aneurysma hinterlegt werden. Voraussetzung ist eine Beerenform mit einem eingeengten Zugang. Die Edelmetallwendel bremst den Blutfluss im Aneurysma, führt zur Bildung eines Thrombus und zur Stilllegung und Abkapselung des stillgelegten Aneurysma. Eine solche Behandlung ist allerdings bei Aneurysmen der Schlagadern nur in seltenen Fällen möglich.

Insbesondere bei Aortenaneurysmen ist es inzwischen auch möglich, so genannte Stentgrafts endovaskulär zu implantieren. Stentgrafts bestehen aus einem distalen und ggf. einem proximalen Stent (vom behandelnden Arzt aus gesehen) sowie einem gewebeverträglichen Schlauchstück aus Kunststoff. Stentgrafts mit zwei Stents werden durch Expandieren des Stents gegen die Gefäßwand verankert, Stentgrafts mit nur einem distalen Stent werden am proximalen Ende mit der Gefäßwand vernäht. Die Methode ist für den Patienten wenig belastend, jedoch sind solche Implantate bislang noch verbesserungsbedürftig, da es immer wieder zu Leckagen kommt. Durch die Leckagen kommt das Aneurysma wieder unter systemischen Druck, die Gefahr der Ruptur besteht weiter. Ferner ist die endovaskuläre Platzierung derartiger Stentgrafts mit herkömmlicher Kathetertechnik verbesserungswürdig.

Ein Problem dieses Stentgrafts ist die Fertigung der Stents aus einem selbstexpandierenden Material, etwa Nitinol, was nicht immer einen hinreichend dichten Abschluss gewährleistet. Auch das Kunststoffgewebe ist nicht immer dicht, so dass Mikroleckagen auftreten. Spezielle Implantate, die für die Aortenbifurkation entwickelt wurden und entsprechend mit drei Stents eine Y-Form haben, haben das Problem, dass der Seitenarm auf komplizierte Weise im Gefäß angekoppelt werden muss. Auch dies schafft Raum für Undichtigkeiten.

Andererseits sind Stentgrafts eine sehr schonende Methode der Stilllegung eines Aneurysmas, so dass eine Weiterentwicklung dieser Technologie wünschenswert ist.

Aus der WO 02/028316 A2 ist ein Stentgraft für die Behandlung arterieller Stenose bekannt, das aus einem konventionellen Stent mit daran anschließendem Inliner besteht. Der Inliner besteht aus einem biokompatiblen Material, beispielsweise PTFE. Das Stentgraft wird proximal zum Stent mit der Gefäßwand vernäht.

Aus der WO 1999/013808 ist ein Stentgraftsystem bekannt, das für die Implantation in arterielle Bifurkationen geeignet ist. Das Implantat setzt sich aus einem primären Graft mit endseitig angeordneten Stents und einem zentralen Anschlusselement sowie einem zweiten Graftelement mit einem Anschlusselement und einem Stent zusammen, die am Implantationsort in der Bifurkation aneinandergekoppelt werden. Die Koppelung ist aufwendig und erfordert einiges Geschick.

Die EP 0 747 088 A1 beschreibt ein System bestehend aus zwei Ballonen, die durch ein Graft verbunden sein können. Eine Verzweigung ist nicht vorgesehen.

Die US 2007/0142819 A1 offenbart einen Bifurkationskatheter mit einem verzweigten Schaft und drei Ballonen, der dazu bestimmt ist, ein Blutgefäß im Bereich einer Verzweigung zu blockieren, um lokal beispielsweise ein Medikament zu applizieren. Der bekannte Bifurkationskatheter weist für jeden der distalen Ballons ein separates Führungsdrahtlumen auf, es ist demnach keine sich verzweigende Führung vorgesehen, was den Katheter im Durchmesser notwendig vergrößert.

Ziel der Erfindung ist die Bereitstellung eines Systems zur Platzierung eines Stentgrafts, das insbesondere im Bereich von Gefäßverzweigungen eingesetzt werden kann, etwa in der Aortabifurkation, über die notwendige Anzahl von Ballonen zur Verankerung der Stents des Grafts an der Gefäßwand verfügt und eine sichere Platzierung des Stentgrafts erlaubt. Dabei sollte der Durchmesser des Systems möglichst klein sein. Das Stentgraft soll über eine einheitliche, lecksichere und gut körperverträgliche schlauchartige Verbindung zwischen den Stents verfügen, die mit den Stents hinreichend fest verbunden ist.

Dieses Ziel wird mit einem System der eingangs bezeichneten Art erreicht, bei dem die Lumina für den Führungsdraht und den oder die distalen Ballone durch den proximalen Ballon hindurchgeführt sind und das Stentgraft auf die Ballone aufgekrimpt ist.

Das erfindungsgemäße System verfügt über zwei distale und einen proximalen Ballon. Solche dreiarmigen Stentgrafts können insbesondere im Bereich der Aortenbifurkation appliziert werden.

Der erfindungsgemäße Ballonkatheter weist separate Lumina zur Beaufschlagung der einzelnen Ballone mit hydraulischem Druck auf. Dies erlaubt die sequenzielle Applikation der Stents des Stentgrafts, wobei zunächst die distalen Stents des Grafts platziert werden und anschließend, unter Spannung des Schlauchteils, der proximale Stent verankert wird. Hierzu werden Lumina für die distalen Stents durch den proximalen Stent hindurchgeführt, ebenso wie das Lumen für den Führungsdraht. Sind zwei distale Stents vorhanden, ist es sinnvoll, dass sich das Lumen für den Führungsdraht verzweigt, so dass der Führungsdraht individuell in die beiden distalen Ballone geführt werden kann. Im Allgemeinen ist das Lumen für den Führungsdraht distal zu den distalen Ballonen geschlossen, so dass über den Führungsdraht eine gewisse Steuerung der Ballone, bis hin zur Ausübung eines gewissen Drucks/Zugs bei der Einspannung des Grafts, möglich ist.

Bei den Stents des Stentgrafts handelt es sich um übliche Stents, wie sie mit Ballonen oder selbstexpandierend in Blutgefäße eingesetzt werden können. In der Regel sind sie aus medizinischem Stahl oder aus Nitinol gefertigt.

Das schlauchförmige Element der Stentgrafts ist ein Faservlies aus elektroversponnenen Mikro- oder Nanofasern. Solche Faservliese sind dem Fachmann grundsätzlich bekannt und beispielsweise in WO 02/49536 A2 und WO 03/045875 A1 beschrieben.

Die Faservliese gemäß WO 02/49536 A2 können aus mehreren Lagen elektroversponnener Mikrofasern bestehen und sind hinsichtlich ihrer Durchlässigkeit für Körperflüssigkeiten optimiert.

Als Materialien für das Faservlies kommen grundsätzlich alle elektroverspinnbaren Polymere in Frage. Insbesondere sind dies aber Polyurethan, Polyester und/oder Polytetrafluorethylen (PTFE), ggf. auch biodegradierbare Kunststoffe, etwa Polylactide oder -glycolide oder Mischpolymere davon.

Das zum Einsatz kommende Faservlies wird vorzugsweise mehrlagig gestellt. Insbesondere ist dieses Faservlies dreilagig ausgebildet. Dabei können die einzelnen Lagen des Faservlieses aus dem gleichen Material bestehen, aber auch unterschiedliche Materialien beinhalten, etwa eine Kombination aus Polyurethan- und PTFE-Mikro- oder -Nanofasern.

Das Stentgraft ist vorzugsweise Y-förmig ausgebildet, d.h. es verfügt über drei mit Stents versehenen Enden. Somit eignet sich das Stentgraft für die Implantierung in Gefäßbifurkationen, insbesondere auch in die Aortenbifurkation, in deren Bereich häufig abdominale Aortenaneurysmen auftreten. Ein solches dreiendiges Stentgraft verfügt über zwei distale Stents und einen proximalen Stent, welche durch das schlauchförmige Faservliesgebilde miteinander verbunden sind.

In diesem Zusammenhang sei darauf hingewiesen, dass die Begriffe "distal" und "proximal" in Bezug auf die Führungsrichtung des Katheters, mit dem das Stentgraft implantiert wird, gewählt sind. "Distal" bedeutet demnach, dass der entsprechende Stent im distalen Bereich des Einführungskatheters angeordnet ist, während ein "proximaler" Stent am arztseitigen Ende des Katheters liegt.

Das Faservlies des schlauchförmigen Stentgrafts ist an die endständigen Stents angesponnen. Dabei ist es sinnvoll, den Stent zumindest teilweise in das Faservlies zu integrieren, vorzugsweise vollständig. Im Bereich des Übergangs von Stent auf Graft ist es zweckmäßig, weitere Verstärkungsschichten vorzusehen. Das Einspinnen der Stents in das Faservlies bewirkt eine optimale Festlegung des Vlieses am Stent bei gleichzeitig guter Dichtwirkung gegen die Gefäßwand nach der Expansion.

Die Erfindung betrifft ferner ein System in der applikationsfertigen Form, d.h. ein System mit einem Stentgraft, bei dem die Stents zur Applikation auf Ballone eines Ballonkatheters aufgekrimpt sind. Dabei sind die einzelnen Ballone separat mit Druck beaufschlagbar, so dass bei der Einbringung in ein Gefäß zunächst die distalen Stents platziert werden können und zum Ende der proximale Stent. Die dazu zum Einsatz kommenden Katheter haben zweckmäßigerweise einen Multilumenschaft, die im Übrigen herkömmliche Bauart haben. Im Falle eines Stentgrafts mit zwei distalen und einem proximalen Stent, wie er für die Aortenbifurkation eingesetzt wird, sind selbstverständlich die beiden distalen Stents separat voneinander führbar und platzierbar, wie auch expandierbar. Dies setzt voraus, dass sich der Ballonkatheter distal zum proximalen Stent in zwei Arme mit jeweils einem Ballon verzweigt.

Die Erfindung wird durch die Abbildungen bevorzugter Ausführungsformen und die nachfolgende Beschreibung dieser Abbildung näher erläutert. Von den Abbildungen zeigen:
- Fig. 1:: ein nicht erfindungsgemäßes Stentgraft mit zwei Stents;
- Fig. 2:: einen nicht erfindungsgemäßen Multilumenkatheter für die Platzierung des Stentgrafts gemäß Fig. 1;
- Fig. 3:: ein erfindungsgemäßes Y-förmiges Stentgraft für die Implantation in Gefäßbifurkationen; und
- Fig. 4:: einen erfindungsgemäßen Multilumenkatheter für die Implantation des Stentsgrafts gemäß Fig. 3.

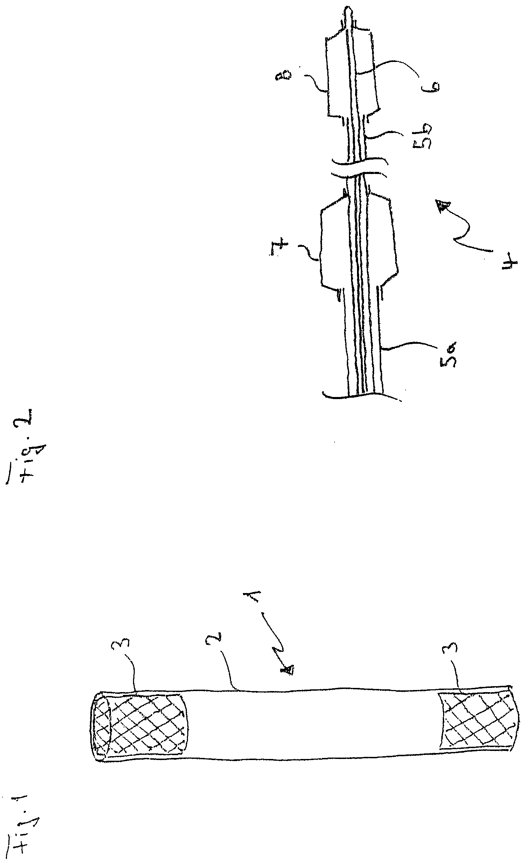

Fig. 1 zeigt ein nicht erfindungsgemäßes Stentgraft 1 mit einem elektrogesponnenen Faservlies 2 und darin integrierten Stents 3 an beiden Enden. Die Stents sind vollständig in das Faservlies eingesponnen, so dass sie oberflächlich mit Faservlies bedeckt sind. Die Elastizität des Faservlieses gewährleistet, dass der Verbund zwischen Faservlies und Stent beim Aufkrimpen des Stents auf einen Ballon erhalten bleibt. Das Gleiche gilt für die Expansion bei der Implantierung in ein Gefäß.

Fig. 2 zeigt schematisch einen nicht erfindungsgemäßen Ballonkatheter 4 mit einem Schaft 5, der sich in einen Multilumenschaft 5a und einen Einfachlumenschaft 5b gliedert. Im Zentrum des Katheters verläuft ein Führungsdrahtlumen 6, in dem der nicht gezeigte Führungsdraht zum Platzieren des Katheters liegt. Ein proximaler Ballon 7 und ein distaler Ballon 8 sind separat über die freien Lumina des Schafts 5 hydraulisch mit Druck beaufschlagbar, so dass ein darauf platziertes Implantat 1 bei dem die Stents 3 auf die Ballone 7 und 8 aufgekrimpt sind, mit Hilfe des Ballonkatheters (Doppelballonkatheters) endoluminal in ein Gefäß platziert werden kann.

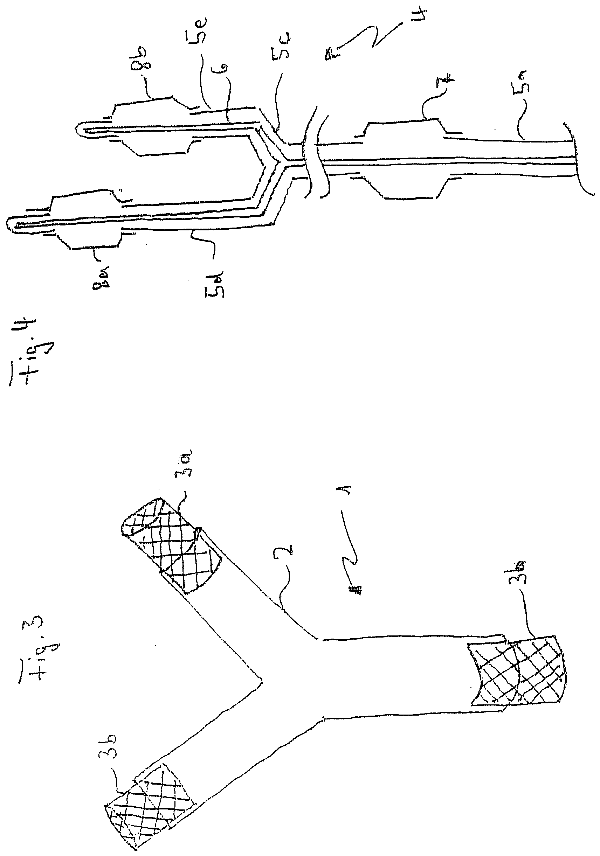

Fig. 3 zeigt ein erfindungsgemäßes Graft mit drei Stents, die jeweils etwa halb in das Faservlies 2 integriert sind. Das Graft hat Y-Form, so dass es in eine Bifurkation des Gefäßsystems implantiert werden kann. Im Falle der Implantation in die Aortenbifurkation ist der Stent 3a, der proximale Stent, der auf den proximalen Ballon des Ballonkatheters gekrimpt wird, während die Stents 3b auf zwei separate Ballone der beiden Arme des Ballonkatheters gemäß Fig. 4 distal zum proximalen Ballon aufgekrimpt werden.

Fig. 4 zeigt schematisch einen Katheter 4 mit einer Verzweigung distal zum proximalen Ballon 7. Der Schaft 5a ist ein Multilumenschaft mit entsprechenden Leitungen zur Beaufschlagung der Ballone 7, 8a und 8b mit hydraulischem Druck. Ein Führungsdrahtlumen 6 durchläuft den Multilumenschaft 5a und gabelt sich im Gabelungsbereich 5c des Schafts und führt in beide Arme 5d und 5e hinein. Der proximale Ballon 7 dient, bei Aufnahme des Implantats gemäß Fig. 3, der Applikation des proximalen Stents 3a, während die distalen Stents 3b auf die Ballone 8a und 8b in den Armen 5d und 5e gekrimpt werden.

Die erfindungsgemäßen Ballonkatheter werden beispielsweise über einen Führungskatheter per femoralem Zugang in die Aorta eingeführt. Bei dem nicht erfindungsgemäßen Graft gemäß Fig. 1 wird der distale Stent oberhalb eines zu behandelnden Aortenaneurysmas positioniert und der Stent dort expandiert. Damit ist der Stent und das damit verbundene Graft distal fixiert. Durch leichten Zug am System kann das Graft leicht gespannt werden, bevor dann der proximale Stent expandiert wird.

Zur Platzierung des erfindungsgemäßen Y-förmigen Grafts im Raum der Aortenbifurkation ist das Prinzip das Gleiche, nur befindet sich parallel zum distalen Ballon noch ein Katheterarm, der bei Applikation des Systems über das entsprechende Führungsdrahtlumen mit dem Führungsdraht in den gegenüberliegenden Bifurkationsabzweig manövriert werden kann. Die Implantation des Grafts erfolgt ähnlich wie bei der zuerst beschriebenen Variante. Zunächst wird der 1. distale Stent expandiert, dann das Graft über den in den Bifurkationszweig führenden Katheterarm unter Spannung gebracht und der 2. distale Stent dort expandiert. Zuletzt wird wieder der proximale Zweig des Grafts unter Spannung gebracht und der proximale Stent expandiert.

Es versteht sich, dass der erfindungsgemäße Ballonkatheter dahingehend modifiziert werden kann, dass mehr als nur eine Abzweigung im Graft vorhanden ist. Beispielsweise kann ein entsprechendes Graft sich auch in drei Arme verzweigen. Es versteht sich ferner, dass derartige Grafts nicht nur für Aorten und die Aortabifurkation einsetzbar sind, sondern auch in anderen Gefäßbereichen.

## Patentansprüche

1. System zur Platzierung von Stentgrafts (1) in einer Gefäßbifurkation bestehend aus einem Stentgraft (1) und einem Ballonkatheter (4), wobei der Ballonkatheter (4) einen proximalen (7) und zwei distale Ballone (8), einen Multilumenschaft (5a) mit einem Lumen (6) für die Aufnahme eines Führungsdrahts und wenigstens drei Lumina (5a, 5b) zur individuellen Beaufschlagung der Ballone (7, 8) mit hydraulischen Druck, aufweist, wobei der Stentgraft (1) einen proximalen (3a) und zwei distale Stents (3b) aufweist, wobei die Stents (3a, 3b) durch ein schlauchförmiges textiles Element (2) miteinander verbunden sind, das eine Y-Form aufweist und die Stents des Stentgrafts (1) auf die Ballone (7, 8) aufgekrimpt sind, die Lumina (6, 5b) für den Führungsdraht und die distalen Ballone (8) durch den proximalen Ballon (7) hindurchgeführt sind und der Ballonkatheter und das Lumen (6) zur Aufnahme des Führungsdrahts distal zum proximalen Ballon (7) Y-förmig verzweigt sind.

2. System nach Anspruch 1, wobei es sich bei dem Stentgraft (1) um einen Aorten-Bifurkationsgraft (1) handelt.

3. System nach Anspruch 1 oder 2, **dadurch gekennzeichnet, dass** das textile Element des Stentgrafts (1) ein Faservlies aus elektroversponnenen Mikro- oder Nanofasern ist und durch Anspinnen an den Stents gefertigt ist.

4. System nach Anspruch 3, **dadurch gekennzeichnet, dass** das Faservlies mehrlagig ist.

5. System nach einem der Ansprüche 3 oder 4, **gekennzeichnet durch** ein dreilagiges Faservlies.

6. System nach einem der Ansprüche 3 bis 5, **dadurch gekennzeichnet, dass** das Faservlies aus Polyurethan-, Polyester- und/oder Polytetrafluorethylenfasern besteht.

7. System nach Anspruch 6, **dadurch gekennzeichnet, dass** das Faservlies aus Polyethylenterephthalatfasern besteht.

8. System nach Anspruch 4 oder 5, **dadurch gekennzeichnet, dass** das mehrlagige Faservlies Lagen aus verschiedenen Fasermaterialien enthält.

9. System nach einem der Ansprüche 3 bis 8, **gekennzeichnet durch** eine Verstärkung des Faservlieses im Anbindungsbereich der Stents (3, 3a, 3b).

10. System nach einem der Ansprüche 3 bis 9, **dadurch gekennzeichnet, dass** die Stents (3, 3a, 3b) zumindest partiell in das Faservlies eingesponnen sind.

## Claims

1. System for the placement of stent grafts (1) in bifurcational lesions comprising a stent graft (1) and a balloon catheter (4), the balloon catheter (4) having one proximal (7) and two distal balloons (8), a multi-lumen shaft (5a) having one lumen (6) to accommodate a guidewire and at least three lumina (5a, 5b) for individually applying hydraulic pressure to the balloons (7, 8), in which the stent graft (1) comprises one proximal (3a) and two distal stents (3b), the stents (3a, 3b) being connected with each other through a Y-shaped textile element (2), and the stents of the stent graft (1) being crimped onto the balloons (7, 8), the lumina (5b, 6) accommodating the guidewire and the distally arranged balloons (8) are passed through the proximal balloon (7), and the balloon catheter and the lumen (6) accommodating the guide wire provide a Y-shaped branching point distally to the proximal balloon (7).

2. System according to claim 1, in which the stent graft (1) is an aortic bifurcation graft (1).

3. System according to claim 1 or 2, **characterized in that** the textile element of the stent graft (1) is a non-woven fabric of electrospun micro- or nano-fibers and is produced by spinning to the stents.

4. System according to claim 3, **characterized in that** the non-woven fabric is of multi-layer design.

5. System according to claim 3 or 4, **characterized by** a three-layer non-woven fabric.

6. System according to any one of the claims 3 to 5, **characterized in that** the non-woven fabric consists of polyurethane, polyester and/or polytetrafluoroethylene fibers.

7. System according to claim 6, **characterized in that** the non-woven fabric consists of polyethylene terephthalate fibers.

8. System according to claim 4 or 5, **characterized in that** the multi-layer non-woven fabric contains layers made of different fiber materials.

9. System according to any one of the claims 3 to 8, **characterized by** a reinforcement of the non-woven fabric in the application zone of the stents (3, 3a, 3b).

10. System according to any one of the claims 3 to 9, **characterized in that** the stents (3, 3a, 3b) are spun at least partially into the non-woven fabric.

## Revendications

1. Système de placement de greffes d'endoprothèse (1) dans un vaisseau bifurqué se composant d'une greffe d'endoprothèse (1) et d'un cathéter à ballonnets (4), dans lequel le cathéter à ballonnets (4) présente un ballonnet proximal (7) et deux ballonnets distaux (8), une tige à lumières multiples (5a) avec une lumière (6) destinée à recevoir un fil de guidage et au moins trois lumières (5a, 5b) destinées à l'alimentation individuelle des ballonnets (7, 8) avec une pression hydraulique, dans lequel la greffe d'endoprothèse (1) présente une endoprothèse proximale (3a) et deux endoprothèses distales (3b), dans lequel les endoprothèses (3a, 3b) sont reliées l'une à l'autre par un élément textile (2) en forme de tuyau souple, qui présente une forme en Y et les endoprothèses de la greffe d'endoprothèse (1) sont rétractées sur les ballonnets (7, 8), les lumières (6, 5b) pour le fil de guidage et les ballonnets distaux (8) sont guidées à travers le ballonnet proximal (7) et le cathéter à ballonnets et la lumière (6) destinée à recevoir le fil de guidage sont ramifiés en forme d'Y en position distale par rapport au ballonnet proximal (7).

2. Système selon la revendication 1, dans lequel la greffe d'endoprothèse (1) est une greffe aortique bifurquée (1).

3. Système selon la revendication 1 ou 2, **caractérisé en ce que** l'élément textile de la greffe d'endoprothèse (1) est un non-tissé de fibres composé de micro- ou nanofibres électrofilées et est fabriqué par filage sur les endoprothèses.

4. Système selon la revendication 3, **caractérisé en ce que** le non-tissé de fibres présente plusieurs couches.

5. Système selon l'une quelconque des revendications 3 ou 4, **caractérisé par** un non-tissé de fibres à trois couches.

6. Système selon l'une quelconque des revendications 3 à 5, **caractérisé en ce que** le non-tissé de fibres se compose de fibres de polyuréthane, de polyester et/ou de polytétrafluoroéthylène.

7. Système selon la revendication 6, **caractérisé en ce que** le non-tissé de fibres se compose de fibres de téréphtalate de polyéthylène.

8. Système selon la revendication 4 ou 5, **caractérisé en ce que** le non-tissé de fibres à plusieurs couches se compose de différents matériaux fibreux.

9. Système selon l'une quelconque des revendications 3 à 8, **caractérisé par** un renforcement du non-tissé de fibres dans la région de fixation des endoprothèses (3, 3a, 3b).

10. Système selon l'une quelconque des revendications 3 à 9, **caractérisé en ce que** les endoprothèses (3, 3a, 3b) sont au moins en partie intégrées par filage dans le non-tissé de fibres.
